# EUROPÄISCHE PATENTSCHRIFT

(11) **EP 0 502 918 B1**
(45) Veröffentlichungstag und Bekanntmachung des Hinweises auf die Patenterteilung: **25.05.1994**
(21) Anmeldenummer: 90917743.8
(22) Anmeldetag: 29.11.1990
(51) Int. Cl.: A61N 1/36

(54) **HERZSCHRITTMACHER**
ARTIFICIAL CARDIAC PACEMAKER
STIMULATEUR CARDIAQUE INTRACORPOREL

(30) Priorität: 29.11.1989 DE 3939899
(43) Veröffentlichungstag der Anmeldung: 16.09.1992
(73) Patentinhaber: BIOTRONIK Mess- und Therapiegeräte GmbH & Co Ingenieurbüro Berlin, D-12359 Berlin (DE)
(72) Erfinder: SCHALDACH, Max, D-8520 Erlangen (DE)
(74) Vertreter: Christiansen, Henning, Dipl.-Ing.
(86) Internationale Anmeldenummer: DE9000929
(87) Internationale Veröffentlichungsnummer: WO9108020

(56) Entgegenhaltungen:
- EP-A- 0 392 800
- WO-A-82/03780
- DE-A- 3 709 022
- US-A- 4 381 552

## Beschreibung

Die Erfindung betrifft einen Herzschrittmacher nach dem Oberbegriff des Anspruchs 1.

Aus "Biomedizinische Technik, 34(1989), 191-196" ist ein Herzschrittmacher bekannt, der für die frequenzadaptive Ein- und Zweikammerstimulation entwickelt wurde und dessen Ratensteuerung auf der Grundlage der Messung des tieffrequenten Schwingungsspektrum als die Aktivität des Patienten kennzeichnender signale erfolgt. Dazu werden die Signale pegellimitiert und frequenzselektiv verstärkt und der Spektralanteil für die körperliche Belastung ausgewertet.

Mit Hilfe eines im Schrittmachergehäuse integrierten piëzoelektrischen Wandlers sowie einer nachgeschalteten Signalverarbeitungschaltung lassen sich die durch die körperliche Belastung auftretenden Bewegungsgrößen erfassen und eine optimale physiologische Anpassung des Schrittmachers an den gegebenen Belastungszustand des Patienten erzielen.

Die dem im Schrittmachergehäuse integrierten piëzoelektrischen wandler nachgeschaltete Signalverarbeitung erfordert jedoch einen zusätzlichen Strombedarf zu den übrigen Schaltungsteilen des Schrittmachers unabhängig vom Lebensrhythmus des Patienten.

Der Erfindung liegt die Aufgabe zugrunde, einen Herzschrittmacher anzugeben, welcher sich an sich wiederholende Aktivitäten des Patienten anpaßt und insbesondere in voraussehbaren Zyklen verminderter körperlicher Aktivität des Patienten auch den Energiebedarf des Schrittmachers herabsetzt.

Diese Aufgabe wird bei einem Herzschrittmacher nach dem Oberbegriff des Anspruchs 1 durch die im Kennzeichen angegebenen Merkmale gelöst.

Auf die parallele Anmeldung Nr. 90 917 162.1, Veröff- Nr. WO 91/08019, wird hingewiesen.

Der Erfindung liegt die Erkenntnis zugrunde, daß der Tagesrhythmus eines Patienten meist zyklisch abläuft und sich somit Möglichkeiten ergeben, insbesondere auch die Schrittmacheraktivität diesen Zyklen anzupassen, so daß insbesondere bei verminderter Aktivität auch die Baugruppen zur Erfassung und Anpassung der Herzrate an diese Aktivität mit verminderter Energie betrieben werden können, da Herzratenänderungen nicht oder nur sehr selten veranlaßt werden müssen.

Hierbei kann die Erkennung der Aktivität des Patienten entweder durch einen von dem Sensor zur Ermittlung der körperlichen Belastung für die Steuerung der Herzrate getrennten Sensor erfolgen, wie beispielsweise durch einen Lagesensor für die Ermittlung der Position des Patienten im Raum (Liegen oder Stehen) oder aber durch den Sensor für die körperliche Belastung selbst. In diesem Fall wird bevorzugt die durchschnittliche Frequenz der Ermittlung der augenblicklichen Belastung mit der Belastung vergrößert, so daß in Ruhephasen nur eine langsamere Anpassung erfolgt.

Die Baugruppe zur Veränderung der Herzrate kann während einer Ruhephase gegebenenfalls ganz abgeschaltet werden. Wird die Herzrate über einen Sensor zur Bestimmung der Leistungsfähigkeit des Patienten ermittelt, der von dem Aktivitätssensor getrennt ist, so kann dieser in einer Ruhephase ebenfalls mit abgeschaltet werden. Im anderen Fall genügt ein regelmäßiges kurzzeitiges Einschalten in relativ großen Zeiträumen.

Zur Ermittlung der Ruhephase des Patienten wird eine Schaltung herangezogen, welche die Periodizität der regelmäßigen Ruhephasen des Patienten ermittelt und daraus ein Steuersignal ableitet, welches eine Verringerung des Energieverbrauchs der die Stimulationsrate des Schrittmachers beeinflussenden Baugruppe veranlaßt. Damit kann dann gegebenenfalls auch die Wiederholungsrate des kurzzeitigen Wiedereinschaltens der Mittel zur Aktivitätserfassung während der regelmäßigen Ruhephasen herabgesetzt bleiben.

Andere Weiterbildungen und vorteilhafte Ausgestaltungen der Erfindung ergeben sich aus den abhängigen Ansprüchen, der weiteren Beschreibung und der Zeichnung.

Um zu vermeiden, daß das relativ stromaufwendige Auswerteprogramm der nachgeschalteten Signalverarbeitungsschaltung eines Aktivitätssensorsystems ohne Unterbrechung aktiviert ist, wird bevorzugt eine Schaltung verwendet, welche den periodischen Rhythmus von Aktivitäts- und Ruhephasen des Patienten erkennt und die Energieversorgung der Schaltung zur aktivitätsabhängigen Steuerung der Herzrate nur während der Aktivitätsphasen auf den höchsten Wert schaltet. Vorzugsweise erfolgt auf diese Weise eine Anpassung an einen Tag/Nacht-Rhythmus (Aktivitäts- und Ruhephasen) des Patienten im Hinblick auf das Zuschalten des Aktivitätssensorsystems während der Tagphase eines Patienten bzw. das Abschalten während der Nachtphase.

Wird ein einziges Aktivitätssensorsystem sowohl für die Steuerung der Herabsetzung der Energieversorgung als auch für die Ratensteuerung benutzt, so ist es günstig, wenn in Zeiten erhöhter Aktivität (Aktivitätsphase) die Häufigkeit der Aktivierung der Schaltung zur Aktivitätserkennung (und damit der Energieverbrauch) heraufgesetzt bzw. deren Ansprechpegel herabgesetzt ist, während in Zeiten niedriger Aktivität (Ruhephase) die Zahl der Aktivierungen der Schaltung pro Zeiteinheit (und somit auch der Energieverbrauch) herabgesetzt ist. Bei der zyklischen Steuerung in Abhängigkeit von den regelmäßigen Aktivitäts- und Ruhephasen des Patienten im 24-Stunden-Rhythmus wird diese "aktivitätsabhängige Aktivität"-Steuerung der Aktivitätserkennungsschaltung nur in den gemittelten Ruhephasen des Patienten ausgenutzt.

Durch die erfindungsgemäße Lösung wird auf vorteilhafte Weise sichergestellt, daß nur dann die Schaltungsmittel zur Aktivitätserkennung mit erhöhter Energie versorgt werden, wenn der Patient sich in einer Aktivitätsphase befindet und eine schnelle Anpassung der Herzstimulationsrate an Belastungsänderungen erforderlich ist. Einzelne Bewegungen, wie sie beispielsweise während der Schlafphase vorkommen, führen somit nicht zu einer Umschaltung auf eine erhöhte Stimulationsrate des Herzschrittmachers.

Als vorteilhaft wird eine Lösung vorgeschlagen, die sich den ändernden Tag/Nachtrhythmen des Patienten, wie sie beispielsweise bei der Umstellung von Sommer- auf Winterzeit oder bei Flugreisen mit Zeitdifferenz eintreten sowie einem von eins verschiedenen Tag/Nacht-Verhältnis anpaßt. Dazu wird insbesondere ein phasenfolgendes System eingesetzt, wie beispielsweise ein PLL-System mit seiner Anpassungsfähigkeit an die Frequenz und Phasenlage sich ändernder Referenzsignale. Als Bezugsphasenlage dient dabei beispielsweise der Übergang von der Aktivitäts- in die Ruhephase, wobei dann die Länge der Ruhephase in einem integrierenden Speicher für Zeitmittelwerte festgehalten wird.

Bei einer anderen vorteilhaften Lösung wird ein Ereigniszähler verwendet, welcher für Aktivitätsphasen kennzeichnende Signale in einem in einem 24-Stunden-Rhythmus zyklisch adressierbaren Mittelwertspeicher jeweils im aktuell adressierten Speicherplatz festhält, wobei die Anzahl der in früheren 24h-Zyklen im Mittel aufgenommenen Aktivitätsereignisse gleichzeitig ausgelesen wird. Diejenigen Speicherplätze, welche im Mittel eine einen vorgegebenen Mindestwert überschreitende Anzahl von Aktivitätsereignissen festhalten, erzeugen beim Auslesen zu einem aktuellen Zeitpunkt dabei ein einen Aktivitätszustand anzeigendes Signal.

Auf diese Weise erhält der Schrittmacher eine Art "innerer Uhr" welche ihn befähigt, seine eigene Tätigkeit an die des Patienten anzupassen. So wird insbesondere die Wiederholrate bei zur Energieersparnis nur in Zeitabständen durchgeführten Messungen oder Berechnungen in der Ruhephase des Patienten weiter herabgesetzt.

Insbesondere läßt sich auch eine Schaltung, welche die Anzahl der Messungen oder vom Prozessor ausgeführten Berechnungen an die augenblickliche Aktivität des Patienten anpaßt, in ihrer Funktion auf die Ruhephasen des Patienten beschränken. Auf diese Weise erfolgt die Anpassung der Herzrate an die körperliche Belastung in der regelmäßigen Aktivitätsphase (also gewöhnlich tagsüber) relativ schnell, während in der Nachtphase, in der Belastungsänderungen in der Regel nicht zu erwarten sind, eine verzögerte Anpassung vorgenommen wird. Dies entspricht aber durchaus dem Verhalten des Patienten selbst, der in einem solchen Fall ebenfalls eher "schlaftrunken" reagieren wird. Erst wenn der Patient regelmäßig zu bestimmten (zunächst ungewöhnlichen Zeiten) eine Aktivitätsphse entwickelt, stellt sich das System aufgrund seiner zeitlichen Anpassungsfähigkeit (Lernfähigkeit) auf diese Regelmäßigkeit ein.

Zur Detektion des Tag/Nacht-Rhythmus bzw. auch eines anderen sich ändernden beliebigen Wechsels zwischen Aktivitäts- und Ruhephasen des Patienten kann dabei insbesondere ein Sensorsystem verwendet werden, welches Aktivitäten des Patienten detektiert und bei Detektion die Herzratensteuerung einschaltet.

Vorteilhafte Weiterbildungen der Erfindung sind in den Unteransprüchen gekennzeichnet bzw. werden nachstenend zusammen mit der Beschreibung der bevorzugten Ausführung der Erfindung anhand der Figuren näher dargestellt. Es zeigen:
Fig. 1 ein Diagramm des zeitlichen Verlaufs der Aktivität eines Patienten im 24h-Rhythmus,
Fig. 2 eine Auswerteschaltung als Ausführungsbeispiel der Erfindung sowie
Fig. 3 ein Blockschaltbild eines mit den erfindungsgemäßen Maßnahmen ausgestatteten ratengesteuerten Herzschrittmachers.

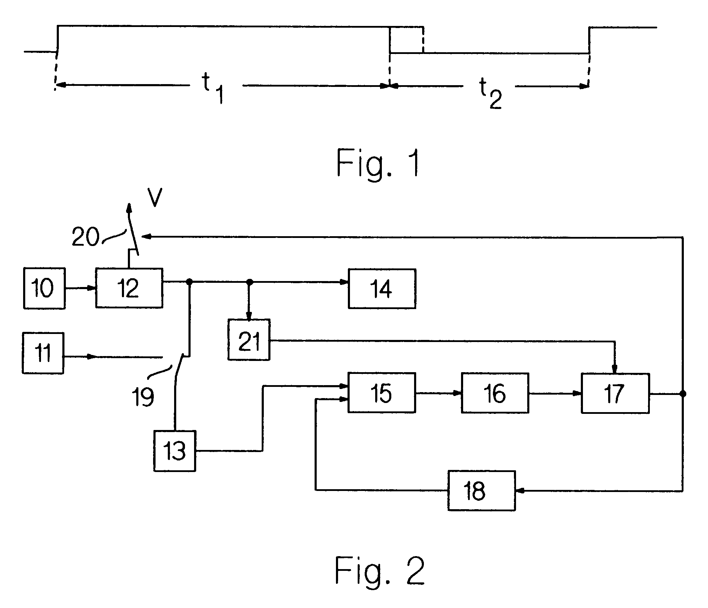

In Figur 1 soll zunächst anhand eines Diagramms der Wechsel zwischen den verschiedenen Aktivitätsphasen des Patienten dargestellt werden. Die Figur zeigt die Tag/Nachtaktivität eines Patienten mit t1 als aktive Zeit und t2 als Ruhezeit. Der Beginn der aktiven Zeit t1 kann sich durch äußere Einflüsse wie Sommer/Winterzeit oder unterschiedliche Zeitzone verändern. Ebenfalls unterliegt das Tast-Verhältnis von Tag zu Nacht Veränderungen. Diese Veränderungen werden durch eine sogenannte "innere Uhr" eines Menschen erfaßt.

Fig. 2 zeigt eine Auswerteschaltung für die Veränderungen der Tag/Nachtaktivität, die hieraus ein Steuersignal für das Abschalten eines Ratensteuersystems 12 zur Energieeinsparung ableitet. Das Aktivitätskriterium kann von einem Aktivitätssensor 10 des Ratensteuersystems 12 abgeleitet werden. Bei dem Ratensteuersystem kann es sich um jedes System handeln, welches die Schrittmacherrate der augenblicklichen Belastung des Patienten nachführt, also beispielsweise um ein aufgrund der Erfassung der systolischen Intervalle oder einer Aktivitätsauswertungarbeitendes System oder ein System zur Umsetzung der ermittelten Respirationsrate des Patienten in eine für die augenblickliche Belastung des Patienten repräsentative Größe. Dem Ratensteuersystem 12 ist ein Herzschrittmacher 14 nachgeschaltet, der in Abhängigkeit von der Belastung des Patienten eine optimale Schrittmacherfunktion erfüllt.

Aus dem Ausgangssignal des Ratensteuersystems 12 wird mittels eines Dekoders 13 eine Referenzfrequenz für eine PLL-Schaltung gewonnen. Der Dekoder 13 erzeugt einen Fig. 1 entsprechenden Spannungsverlauf der Referenzfrequenz.

Durch einen Umschalter 19 kann die Referenzfrequenz auch aus einem Lagesensor 11 oder einem Bewegungssensor abgeleitet werden. Die Referenzfrequenz wird dann einer Phasenvergleichsschaltung 15 zugeführt. Als zweite Frequenz wird dem Phasenvergleichsschaltung 15 das Ausgangssignal eines spannungsgesteuerten Oszillators 17 über einen Teiler 18 zugeführt. Das Ausgangssignal der Phasenvergleichsschaltung 15 wird über einen Tiefpaß 16 dem spannungsgesteuerten Oszillator 17 zugeführt. Das Ausgangssignal des spannungsgesteuerten Oszillators 17 wird gegebenenfalls über einen nicht gezeichneten Teiler als Steuersignal für einen steuerbaren Schalter 20 verwendet, der während der Tagphase das Ratensteuersystem 12 an die Versorgungsspannung V anschaltet und während der Nachtphase dieses von der Versorgung abtrennt.

Der Schalter 20 kann, statt völlig abgeschaltet zu werden, auch periodisch mit einer verringerten Wiederholrate eingeschaltet werden, um auf diese Weise einer Energieersparnis zu erzielen.

Da das Zeitdauer-Verhältnis von Tag zu Nacht bei einem Patienten unterschiedlich sein kann, ist zusätzlich ein integrierender Speicher vorgesehen, der einen diesem Verhältnis proportionalen Spannungswert erzeugt. Dieser Spannungswert wird vorzugsweise dazu verwendet, das Tastverhältnis der Frequenz des spannungsgesteuerten Oszillators 17 an das Zeitdauer-Verhältnis von Tag zu Nacht anzupassen.

Hierbei wird eine PLL-Schaltung mit einem spannungsgesteuerten Oszillator 17 mit niedriger Frequenz verwendet. Dadurch wird die Periodizität des morgendlichen Erwachens mit großer Wahrscheinlichkeit korrekt vorhergesagt. Das PLL-System erfaßt die Phasenlage des täglichen Anstiegs bzw. Abfalls der körperlichen Aktivität zum Ende bzw. Beginn der täglichen Ruhephase und stellt die Schrittmacheraktivität entsprechend ein. Dabei ist lediglich ein Abgleich der Phasenlage erforderlich, da die 24h-Wiederholungsrate durch den Lauf der Erde um die Sonne - und damit den natürlichen Tag-Nacht-Rhythmus - bestimmt wird und damit für alle Patienten festliegt. Zu kompensierende Phasenverschiebungen ergeben sich aber beispielsweise durch Flugreisen (jet lag). Die vorbeschriebene Schaltung braucht nicht wie eine Uhr gestellt zu werden, da sie sich auch in ihrer 24h-Frequenz dem Tagesrhythmus des Patienten anpaßt, so daß kein hochgenauer Zeitgeber erforderlich ist.

Bei einer anderen Ausführung der Erfindung werden die Baugruppen 15 bis 18 und 21 ersetzt durch einen im 24-h-Rhythmus zyklisch adressierten Speicher, der für jedes einer Anzahl von zeitintervallen jeweils einen Speicherplatz aufweist. Die aktuellen Aktivitätsereignisse werden dem gespeicherten Mittelwert für das aktuell adressierte Zeitintervall bei der Mittelung hinzugefügt, während der beim vormaligen Erreichen des betreffenden dem Zeitintervall zugeordneten Speicherplatz vorhandene Speicherinhalt ausgelesen wird und das Ausgangssignal den Schalter V ansteuert, d.h. bei einem zu einer erhöhten Aktivität gehörenden Speicherwert schließt oder mit einer höheren Wiederholrate schließt, während bei einem zu einer niedrigen Aktivität gehörenden Speicherinhalt der Schalter 20 geöffnet bzw. mit niedrigerer Frequenz geschlossen wird, um den Energiespeicher des Schrittmachers vor vorzeitiger Erschöpfung zu bewahren.

Auf diese Weise erkennt der Schrittmacher den Lebensrhythmus des Patienten und paßt das Stimulationsverhalten wie eine "innere Uhr" entsprechend an, ohne daß es dazu zusätzlicher Meßwertaufnehmer bedarf.

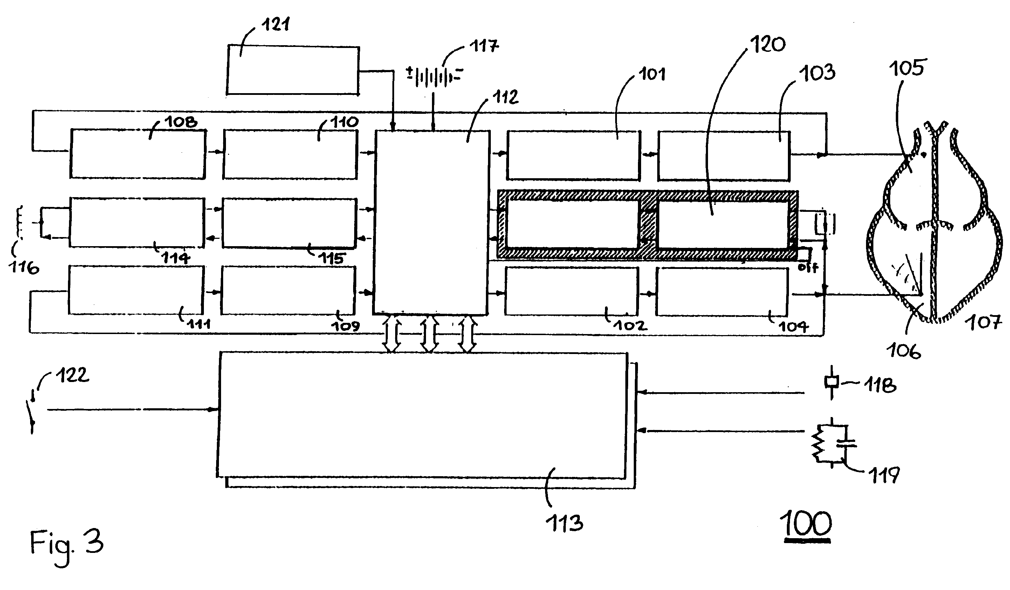

In Figur 3 ist ein ratengesteuerter Herzschrittmacher 100 im Blockschaltbild dargestellt. Dieser Herzschrittmacher stimuliert über Steuerstufen 101 bzw. 102 und Ausgangsstufen 103 bzw. 104 Atrium 105 bzw. Ventrikel 106 des Herzens 107 mittels entsprechender an die Ausgangsschaltungen angeschlossener Elektroden. Diese Elektroden nehmen auch für Herzeigenaktionen charakteristische Signale aus dem Herzen auf und führen diese einer Eingangsverstärker-Schaltung 108 bzw. 111 zu. Über nachgeschaltete Detektionsstufen 110 bzw. 109 - jeweils getrennt für Atrium und Ventrikel - werden diese Signale aufbereitet.

Diese Daten werden einem Prozessorsystem 112 zugeleitet. Dieses Prozessorsystem ist mit der Steuerlogik über einen Daten-, einen Steuer- und einen zusätzlichen Bus verbunden, so daß von der Prozessoreinheit die digitalisierten im Atrium und Ventrikel aufgenommenen Signale abgefragt werden können und weiterhin digitale Steuersignale zur Auslösung von Stimulationsimpulsen im Atrium und Ventrikel abgegeben werden können. Die Steuerlogik 112 verhält sich zum Prozessorteil 113 wie ein Steuerteil für eine periphere Einheit, beispielsweise als Interface zur Aufnahme und Abgabe von externen Analogsignalen mit AD-DA-Wandler, vielfach auch als Eingangs-Ausgangs-Einheit bezeichnet.

Weiterhin ist mit der Steuerlogik ein Zweirichtungsübertragungskanal 114, 115. verbunden, der mittels einer Induktivität 116 sowohl im Herzen aufgenommene Signale nach außerhalb des Patienten oder aber Steuersignale zur Programmierung des Herzschrittmachers von außerhalb des Patienten her zum Schrittmacher übertragen kann. Weiterhin sind vorgesehen: eine Batterie 117 zur Versorgung des gesamten Schrittmachers, ein Quarz 118 und ein Schwingkreis 119 als Zeitgeber bzw. Reservezeitgeber.

Ein Signalaufnahmeteil 120 dient zur Ermittlung eines physiologischen Parameters innerhalb des Körpers des Patienten, der ein Maß für die körperliche Belastung bildet. Bei dem dargestellten Ausführungsbeispiel handelt sich dabei um einen Impulsgeber zur Abgabe von Meßimpulsen (Stromimpulse i) im rechten Ventrikel, aus denen über die im Ventrikel befindliche Elektrode der aktuelle elektrische Widerstand innerhalb des Ventrikels zu vorbestimmten Zeiten ermittelt werden kann. Weiterhin ist die Schaltung 120 versehen mit einer Leitung zum Abschalten dieser Baugruppe, so daß insbesondere die Abgabe von Stromimpulsen unterbunden wird. Der Block 120 ist mittels eines Signals "off" von der Steuerlogik 112 her abschaltbar. Diese Abschaltung erfolgt vom Prozessorteil 113 her.

Schließlich ist noch der Ruhephasenschalter 121 dargestellt, der Phasen der körperlichen Aktivität bzw. Ruhe des Patienten erfaßt. Der Ruhephasenschalter ist mit dem Prozessorsystem 112 zum direkten Datenaustausch verbunden. Der Ruhephasenschalter 121 enthält die weiter oben dargestellte PLL-Einheit mit Positions- oder Bewegungssensor. Diese ist derart gestaltet, daß das Ausgangssignal des Positions- oder Bewegungssensors mit dem Ausgangssignal der den Aktivitätsrhythmus des Patienten erkennenden Einheit mittels UND-Gatter verknüpft ist. Sporadische Bewegungen oder Lageänderungen des Patienten werden erst dann als Beginn der Aktivitätsphase erkannt, wenn auch der Zyklus der zu erwartenden Aktivitätsphase begonnen hat, so daß verhindert ist, daß der Herzschrittmacher bereits bei einzelnen Lageänderungen, beispielsweise im Schlaf, eine Betriebsweise beginnt, die der aktiven Phase des Patienten (Tagesprogramm) zugeordnet ist. Nur wenn eine intensivere Aktivität des Patienten in einer Phase erkannt wird, in der eigentlich eine Ruhephase zu erwarten wäre, schaltet der Schrittmacher ebenfalls auf sein der aktiven Phase zugeordnetes Programm um. Bei diesem Programm wird dann beispielsweise der Block 121 durch Unterdrückung des Signals "off" aktiviert, so daß die Stimulationsrate adäquat zur körperlichen Belastung verändert wird. Da diese Schaltung wegen der regelmäßig zu erzeugenden Stromimpulse erheblich Batterieenergie verbraucht, ist ihre Benutzung in Ruhephasen des Patienten entbehrlich. Durch die erfindungsgemäße Anordnung wird sichergestellt, daß die Aktivierung nur dann erfolgt, wenn die Bewegungen oder Lageänderungen des Patienten auch tatsächlich zu einer längerfristigen Aktivität gehören.

Mittels eines Reed-Schalters 122 läßt sich die Prozessoreinheit 113 mittels eines externen Magneten in einen vorbestimmten Zustand setzen, in dem vorzugsweise mit einer festen Rate stimuliert wird.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Ratengesteuerter Herzschrittmacher mit einer Baugruppe (12) zur Anpassung der Stimulationsrate an die aktuelle körperliche Belastung eines Patienten sowie einem sensor (10, 11) und einer Schaltung zur Ermittlung der Aktivität des Patienten (13-15, 18)
**dadurch gekennzeichnet**, daß
die Schaltung zur Ermittlung der Aktivität des Patienten aus einer Schaltung (13,15-18) besteht, deren Eingang mit dem Ausgang des Sensors (10,11) für den Aktivitätszustand des Patienten verbunden ist, welcher im Falle über einen längeren Zeitraum fehlender oder geringer Aktivität oder einen vorgegebenen Pegel überschreitender Aktivität jeweils ein Ausgangssignal an den Synchronisationseingang eines in einem, vorzugsweise 24-Stunden-, Rhythmus mit in kontinuierlichem Wechsel auftretende Aktivitäts- und Ruhephasen synchronisierbaren Zeitgebers (17) abgibt, welcher auch unabhängig von einem derartigen Eingangssignal ein eine Ruhe- oder eine Aktivitätsphase anzeigendes Signal erzeugt, wobei der Wechsel zwischen Äktivitäts- und Ruhephase im Mittel dem Wechsel der in einem vorangehenden Zeitraum aufgenommenen Ausgangssignale des Sensors für den Aktivitätszustand (10, 11) folgt.

2. Herzschrittmacher nach einem oder mehreren der Ansprüche 1 bis 4, **dadurch gekennzeichnet**, daß das Ausgangssignal des Sensors (10, 11) die Referenzfrequenz einer PLL-Schaltung (13. 15, 16, 17, 18) oder ein Ereignissignal zur Abspeicherung in einem periodisch in einem 24-Stunden-Zyklus Speicherplätze für Ereignis-signale adressierenden Ereigniszähler als Ausführungsformen des in einem 24-Stunden-Rhythmus synchronisierbaren Zeitgebers bildet.

3. Herzschrittmacher nach Anspruch 2, **dadurch gekennzeichnet**, daß die PLL-Schaltung (13, 15, 16, 17, 18) einen spannungsgesteuerten Oszillator (17) mit niedriger, im Bereich der Frequenz des Aktivitäts-/Ruhe-Rhythmus liegender Frequenz aufweist.

4. Herzschrittmacher nach Anspruch 3, **dadurch gekennzeichnet**, daß aus dem Ausgangssignal des spannungsgesteuerten Oszillators (17) ein Steuersignal zum Ab-bzw. Einschalten der Baugruppe (12) zur Anpassung der Stimulationsrate erzeugt wird.

5. Herzschrittmacher nach Anspruch 4, **dadurch gekennzeichnet**, daß ein integrierender Speicher (21) aus dem Aktivitäts-/Ruhe-Rhythmus einen dem Verhältnis der Aktivitäts- zur Ruhepase des Patienten proportionalen Spannungswert erzeugt, und daß dieser Spannungswert das Tastverhältnis der Frequenz des spannungsgesteuerten Oszillators (17) an das Verhältnis des Aktivitäts- zur Ruhephase des Patienten anpaßt.

6. Herzschrittmacher nach Anspruch 5, **dadurch gekennzeichnet**, daß das Ausgangssignal der Schaltung zur Ermittlung des Aktivitäts-/Ruhe-Rhythmus (13,15-18) mittels UND-Verknüpfung logisch verknüpft ist mit dem Ausgangssignal eines Bewegungs- oder Lagesensors (11), so daß ein die Aktivitätsphase des Patienten kennzeichnendes Signal erst abgegeben wird, wenn ein eine Bewegung- oder Lageänderung des Patienten kennzeichnendes Signal mit einem eine Aktivitätsphse kennzeichnenden Signal des Zeitgebers (17) zusammenfällt.

7. Herzschrittmacher nach einem der vorangehenden Ansprüche, **dadurch gekennzeichnet**, daß eine zu überschreitende Schwelle für die Auswertung der Aktivitätssignale vorgesehen ist, welche während der Ruhephase erhöht ist.

8. Herzschrittmacher nach einem der vorangehenden Ansprüche, **dadurch gekennzeichnet**, daß während der Ruhephase die Anzahl der Einschaltungen der Baugruppe (12) zur Anpassung der Stimulationsrate herabgesetzt ist.

9. Herzschrittmacher nach Anspruch 8, **dadurch gekennzeichnet**, daß während der Ruhephase die Anzahl der Einschaltungen der Baugruppe (12) zur Anpassung der Stimulationsrate dann herabgesetzt ist, wenn die Schaltung zur Ermittlung der Aktivität des Patienten (13,15-18) ein Ausgangssignal abgibt, das anzeigt, daß eine vorgegebene Mindestaktivität für einen vorgegebenen Mindestzeitraum unterschritten ist, oder wenn die Summe solcher Zeiträume innerhalb eines vorgegebenen Zeitintervalls einen vorgegebenen Wert überschreitet.

## Claims

1. A rate-controlled heart pacemaker with an assembly (12) for adapting the stimulation rate to the current physical stress of a patient and with a sensor (10, 11) and a circuit for determining the activity of the patient (13-15, 18), characterized in that the circuit for determining the activity of the patient consists of a circuit (13, 15-18), the input of which is connected to the output cf the sensor (10, 11) for the activity state of the patient, which, in the event of the absence of activity or low activity over a relatively long period of time or activity exceeding a predetermined level, emits a respective output signal to the synchronisation input of a timer (17) which can be synchronised in a, preferably 24-hour, rhythm with activity- and rest phases occurring in continuous alternation, which timer, also independently of such an input signal, generates a signal indicating a rest- or activity phase, where the change-over between activity- and rest phase on average follows the change in the output signals of the sensor for the activity state (10, 11) received in a preceding time period.

2. A heart pacemaker as claimed in one or several of Claims 1 to 4, characterised in that the output signal of the sensor (10, 11) forms the reference frequency of a PLL-circuit (13, 15, 16, 17, 18) or an event signal for storage in an event store, which addresses storage positions for event signals periodically in a 24-hour cycle, as embodiments of the timer which can be synchronised in a 24-hour rhythm.

3. A heart pacemaker as claimed in Claim 2, characterised in that the PLL- circuit (13, 15, 16, 17, 18) comprises a voltage-controlled oscillator (13) with a low frequency lying in the region of the frequency of the activity/rest rhythm.

4. A heart pacemaker as claimed in Claim 3, characterised in that a control signal for de-activating or activating the assembly (12) for adapting the stimulation rate is produced from the output signal of the voltage-controlled oscillator (17).

5. A heart pacemaker as claimed in Claim 4, characterised in that from the activity/rest rhythm an integrating store (21) produces a voltage value proportional to the ratio of the activity- to rest phase of the patient, and that this voltage value adapts the keying ratio of the frequency of the voltage-controlled oscillator (17) to the ratio of the activity- to rest phase of the patient.

6. A heart pacemaker as claimed in Claim 5, characterised in that the output signal of the circuit for determining the activity/rest rhythm (13, 15-18) is logic-linked by an AND function to the output signal of a movement- or position sensor (11) so that a signal characterising the activity phase of the patient is not emitted until a signal characterising a change in the movement or position of the patient coincides with a signal from the timer (17) characterising an activity phase.

7. A heart pacemaker as claimed in one of the preceding claims, characterised in that a threshold to be exceeded is provided for the analysis of the activity signals, which threshold is increased during the rest phase.

8. A heart pacemaker as claimed in one of the preceding claims, characterised in that during the rest phase the number of activations of the assembly (12) for adapting the stimulation rate is reduced.

9. A heart pacemaker as claimed in Claim 8, characterised in that during the rest phase the number of activations of the assembly (12) for adapting the stimulation rate is reduced if the circuit for determining the activity of the patient (13, 15-18) emits an output signal which indicates that a predetermined minimum activity has been undershot for a Predetermined minimum time period or if the sum of such time periods exceeds a predetermined value within a predetermined time interval.

## Revendications

1. Stimulateur cardiaque commandé en taux comportant un sous-ensemble (12) pour l'adaptation du taux de stimulation à l'effort physique actuel d'un patient ainsi qu'un capteur (10, 11) et un circuit pour déterminer l'activité du patient (13-15, 18), caractérisé en ce que le circuit pour déterminer l'activité du patient consiste en un circuit (13, 15-18), dont l'entrée est reliée à la sortie du capteur (10, 11) pour l'état d'activité du patient, qui, dans le cas d'une absence d'activité ou d'une faible activité pendant une durée prolongée ou d'une activité excédant un niveau prédéterminé, délivre à chaque fois un signal de sortie à l'entrée de synchronisation d'un générateur de rythme (17) synchronisable en un rythme, de préférence de 24 h, avec des phases d'activité et des phases de repos qui surviennent en alternance continuelle, lequel produit, même indépendamment d'un tel signal d'entrée, un signal indiquant une phase de repos ou une phase d'activité, l'alternance entre phase d'activité et phase de repos suivant en moyenne l'alternance des signaux de sortie du capteur pour l'état d'activité (10, 11) reçus au cours d'une durée précédente.

2. Stimulateur cardiaque selon la revendication 1, caractérisé en ce que le signal de sortie du capteur (10, 11) forme la fréquence de référence d'un circuit PLL (13, 15, 16, 17, 18) ou un signal d'évènement destiné à la mémorisation dans un compteur d'évènements adressant périodiquement, en un cycle de 24 h, des emplacements de mémoire pour des signaux d'évènements en tant que formes de réalisation du générateur de rythme synchronisable en un rythme de 24 h.

3. Stimulateur cardiaque selon la revendication 2, caractérisé en ce que le circuit PLL (13, 15, 16, 17, 18) comporte un oscillateur commandé en tension (17) à fréquence basse située dans le domaine de la fréquence du rythme activité/ repos.

4. Stimulateur cardiaque selon la revendication 3, caractérisé en ce qu'un signal de commande pour la mise en service ou la mise hors service du sous-ensemble (12) pour l'adaptation du taux de stimulation est produit à partir du signal de sortie de l'oscillateur commandé en tension (17).

5. Stimulateur cardiaque selon la revendication 4, caractérisé en ce qu'une mémoire intégratrice (21) produit à partir du rythme activité/repos une valeur de tension proportionnelle au rapport des phases d'activité et de repos du patient et en ce que cette valeur de tension adapte le rapport d'impulsions de la fréquence de l'oscillateur commandé en tension (17) au rapport des phases d'activité et de repos du patient.

6. Stimulateur cardiaque selon la revendication 5, caractérisé en ce que le signal de sortie du circuit pour déterminer le rythme activité/repos (13, 15-18) est relié de manière logique au moyen d'un circuit ET au signal de sortie d'un capteur de mouvement ou de position (11), de sorte qu'un signal caractérisant la phase d'activité du patient n'est délivré que lorsqu'un signal caractérisant une modification de mouvement ou de position du patient coïncide avec un signal du générateur de rythme (17) qui caractérisé une phase d'activité.

7. Stimulateur cardiaque selon l'une des revendications précédentes, caractérisé en ce qu'il est prévu, pour l'interprétation des signaux d'activité, un seuil à franchir qui est relevé pendant la phase de repos.

8. Stimulateur cardiaque selon l'une des revendications précédentes, caractérisé en ce que, pendant la phase de repos, le nombre des mises en service du sous-ensemble (12) pour l'adaptation du taux de stimulation est réduit.

9. Stimulateur cardiaque selon la revendication 8, caractérisé en ce que, pendant la phase de repos, le nombre des mises en service du sous-ensemble (12) pour l'adaptation du taux de stimulation est réduit lorsque le circuit pour déterminer l'activité du patient (13, 15-18) délivre un signal de sortie qui indique qu'une activité minimale prédéterminée est franchie vers les valeurs inférieures pendant une durée minimale prédéterminée ou lorsque la somme de telles durées excède une valeur prédéterminée à l'intérieur d'un intervalle de temps prédéterminé.
